# EUROPEAN PATENT SPECIFICATION

(11) **EP 4 154 921 B1**
(45) Date of publication and mention of the grant of the patent: **08.11.2023**
(21) Application number: 22198209.3
(22) Date of filing: 27.09.2022
(51) Int. Cl.: A61L 15/24, A61L 15/42, A61L 15/46, A61L 15/50

(54) **ANTI-INFECTIVE AND ANTI-ADHESIVE WOUND DRESSING BASED ON NONSPECIFIC ADHESION TO BACTERIA**
ANTIINFEKTIVER UND ANTIADHÄSIVER WUNDVERBAND AUF DER BASIS VON NICHTSPEZIFISCHER HAFTUNG AN BAKTERIEN
PANSEMENT ANTI-INFECTIEUX ET ANTI-ADHÉSIF BASÉ SUR UNE ADHÉRENCE NON SPÉCIFIQUE À DES BACTÉRIES

(30) Priority: 28.09.2021 CN 202111158438
(43) Date of publication of application: 29.03.2023
(73) Proprietor: ZHENDE MEDICAL CO., LTD., Shaoxing, Zhejiang 312000 (CN)
(72) Inventor: Jianguo, Lu, Shaoxing City, 312000 (CN); Zhentan, Lu, Shaoxing City, 312000 (CN); Huan, Zhang, Shaoxing City, 312000 (CN)
(74) Representative: Loo, Chi Ching

(56) References cited:
- US-A1- 2011 195 624
- US-A1- 2012 263 771
- US-A1- 2017 216 478

## Description

### TECHNICLAL FIELD

The disclosure relates to a technical field of antibacterial materials, in particular to an anti-infective and anti-adhesive wound dressing based on nonspecific adhesion to bacteria.

### BACKGROUND ART

Post-traumatic wound exposure with infection is one of the most common clinical complications, which is also an unavoidable problem in wound treatment. After infection, the wound is difficult to heal for a long time due to insufficient local blood perfusion, malnutrition and tissue hypoxia. If deep infection occurs, it may result in delayed healing or non-healing or other consequences, which may even lead to physical disability or even threaten life.

Bacteria is a main reason for wound infection. After the bacteria invades to the wound, it may adhere to the wound and interact with each other to form bacterial aggregates, and with accumulation of the bacteria, a mature and stable biofilm will be formed. After the biofilm is formed, the bacteria multiplies and secretes toxic factors, which affect function of inflammatory cells and delay wound healing.

Currently, clinical treatment of infected wounds mainly includes surgical debridement, negative pressure wound therapy, antibiotics, antibacterial dressing and other methods. Such operative methods are complicated, which can easily lead to local destruction of the biofilm and release of a large number of bacteria, which may present a risk of acute attack of inflammation or deep invasion of infection. Use of antibiotics is an important means to kill bacteria, but due to physical protection of extracellular matrix in bacterial biofilm and enhancement of drug resistance of the bacterial, bactericidal effect of the antibiotics is greatly reduced. In addition, antibacterial dressings such as silver ions can effectively kill and destroy biofilms and kill bacteria on wounds, but their cytotoxicity to tissues is still in question. Especially, when bacteria fragments enter human tissues, wound inflammation may be aggravated, which is also an unfavorable factor for wound healing. Therefore, if a material can be provided that can effectively adhere to the bacteria to prevent it from spreading to human cells, above problems can be effectively solved, and this is a clean and pollution-free anti-infection mode. However, there is little research on this adhesive wound dressing in related art, and adhesive and anti-infective performance needs to be improved.

In view of this, it is necessary to design an improved anti-infective and anti-adhesive wound dressing based on nonspecific adhesion to bacteria, so as to effectively solve above problems.

US 2017/216478 A1 discloses a wound dressing comprising a fibroin layer made of nanofibers capable of covering a wound while physically removing bacteria from it, without the need of additional substances, e.g. bactericides.

### SUMMARY

In order to overcome shortcomings of the related art, the present disclosure aims to provide an anti-infective and anti-adhesive wound dressing based on nonspecific adhesion to bacteria, which includes a micron-sized porous membrane with polyolefin contained in its surface and does not contain any bactericide. According to the disclosure, a porous membrane with high-efficiency adhesion to wound bacteria is prepared with adaptability of surface energy and a structure of the porous membrane to surface energy of the bacteria, so that both anti-infection for the bacteria and the anti-adhesion to the wound can be realized, with low production cost and ease of popularization and application.

In order to realize above objectives, an anti-infective and anti-adhesive wound dressing based on nonspecific adhesion to bacteria is provided in the present disclosure, which includes a micron-sized porous membrane with polyolefin contained in a surface thereof and does not contain any bactericide. The micron-sized porous membrane forms nonspecific adhesion to the bacteria on the wound by adaptability of its own surface energy and a structure of the porous membrane to the surface energy of the bacterial, so as to realize the anti-infection for the bacteria and the anti-adhesion to the wound; wherein the polyolefin is polymethylpentene.

As a further improvement of the disclosure, the polyolefin on the surface of micron-sized porous membrane is compounded by grafting or coating on a substrate surface of the micron-sized porous membrane, or the micron-sized porous polyolefin fiber membrane is directly obtained by polyolefin spinning.

As a further improvement of the disclosure, a micron-sized porous membrane substrate is a micron-sized porous fiber membrane substrate.

As a further improvement of the disclosure, a pore diameter of the micron-sized porous membrane is 1 to 10 µm.

As a further improvement of the present disclosure, fiber diameters of the micron-sized porous fiber membrane substrate and the micron-sized porous polyolefin fiber membrane are 0. 1 to 15 µm.

As a further improvement of the disclosure, a preparation method of a micron-sized porous polyolefin fiber membrane includes: preparing a polyolefin spinning solution with a mass fraction of 1% to 7%, and then carrying out electrostatic spinning so as to receive and obtain the micron-sized porous polyolefin fiber membrane with an aluminum foil. A solvent of the polyolefin spinning solution includes cyclohexane and N, N dimethylformamide.

As a further improvement of the disclosure, the solvent of the polyolefin spinning solution also includes acetone.

As a further improvement of the disclosure, mass fractions of cyclohexane, N, N dimethylformamide and acetone in the spinning solution are in ranges of 70% to 80%, 10% to 20%, and 0% to 20% respectively.

As a further improvement of the disclosure, an extrusion speed of the electrostatic spinning is 1 mL/h to 7 mL/h; a distance for receiving the fiber is 8 to 12 cm; a voltage for the electrostatic spinning is +18 kV to +20 kV, or -1 kV to -3 kV; and a spinning temperature is 45°C to 60°C.

The disclosure includes following beneficial effects.
1. The anti-infective and anti-adhesive wound dressing based on nonspecific adhesion to bacteria according to the disclosure is a micron-sized porous membrane material with polyolefin contained in its surface, with no bactericide being added. In this way, nonspecific adhesion to the bacteria on the wound can be formed by good adaptability of the surface energy of the micron-sized porous membrane and a structure of the porous membrane to the surface energy of the bacterial, preventing the bacteria from penetrating into wound tissues and thus realizing the anti-infection for the bacteria and the anti-adhesion to the wound. When a porous poly-4-methyl-1 pentene fiber membrane is selected, the anti-infective performance and the anti-adhesive performance to the wound are optimal, which is superior to that of similar dressings in the market.
2. With the anti-infective and anti-adhesive wound dressing based on nonspecific adhesion to bacteria according to the disclosure, the wound bacteria can be transferred and removed by changing dressings by using bacterial adhesion of the micron-sized porous membrane material to adhere to the wound bacteria, thus reducing concentration of the wound bacteria and further reducing a risk of wound infection. Therefore, compared with general anti-infective dressings, no antibacterial ingredient is required to be added, and the dressing of the present disclosure has good biocompatibility and thus no negative impact on the wound to inhibit its recovery, which breaks through inherent antibacterial thought of anti-infective dressings; and high-efficiency anti-infective and anti-adhesive performance can be achieved through targeted selection and design of a material and a surface structure of the porous membrane.
3. For the anti-infective and anti-adhesive wound dressing based on nonspecific adhesion to bacteria according to the disclosure, the micron-sized porous polyolefin fiber membrane can be directly prepared by an electrostatic spinning method or can be obtained through spinning to obtain fibers followed by weaving, so as to obtain the required anti-infective dressing without post-treatment and addition of any bactericide. Therefore, it is with a simple preparation method, low production cost, excellent anti-infective performance, which facilitates popularization and application.

### BRIEF DESCRIPTION OF THE DRAWINGS

FIG. 1 is a scanning electron microscope image of a porous fiber membrane of poly-4-methyl-1 pentene prepared in Embodiment 1 at different magnifications;
FIG. 2 shows data of adhesion performance to bacterial of an anti-infective dressing prepared in Embodiments 1-8;
FIG. 3 shows treatment and healing processes of infected wounds of rats in groups a, b, c, and d, namely, a bacterial infected group (with no treatment), an infected and treated group (with the anti-infective dressing in Comparative Embodiment 1), an infected and treated group (with the anti-infective dressing in Embodiment 1) and a blank control group (with no infection), respectively.
FIG. 4 shows survival rates in treatment and healing processes of infected wounds of rats in groups a, b, c, and d, namely, a bacterial infected group (with no treatment), an infected and treated group (with the anti-infective dressing in Comparative Embodiment 1), an infected and treated group (with the anti-infective dressing in Embodiment 1) and a blank control group (with no infection), respectively.

### DETAILED DESCRIPTION

In order to make objects, technical schemes and advantages of the present disclosure more clear, the present disclosure will be described in detail below with reference to specific examples.

Here, it should also be noted that, in order to avoid obscuring the present disclosure with unnecessary details, only structures and/or processing steps closely related to the schemes of the present disclosure are shown in specific embodiments, while other details irrelevant to the present disclosure are omitted.

In addition, it should be noted that terms "comprising", "including" or any other variation thereof are intended to encompass a non-exclusive inclusion, so that a process, method, article or apparatus including a series of elements includes not only those elements, but also other elements not explicitly listed, or elements inherent to such process, method, article or apparatus.

An anti-infective and anti-adhesive wound dressing based on nonspecific adhesion to bacteria is provided in the present disclosure, which includes a micron-sized porous membrane with polyolefin contained in a surface thereof and does not contain any bactericide, wherein the polyolefin is polymethylpentene. The micron-sized porous membrane forms nonspecific adhesion to the bacteria on the wound by adaptability of its own surface energy and the structure of the porous membrane to surface energy of the bacterial, so as to realize the anti-infection for the bacteria and the anti-adhesion to the wound. It is shown in this disclosure that the micron-sized porous membrane with polyolefin contained in a surface thereof can be prepared to be directly used for anti-infection of wound bacteria. Compared with the prior art, no bactericide is required to be added, and with adhesion of polyolefin on the surface to the bacteria, the bacteria can be prevented from penetrating into wound cells. Moreover, it does not occur that inflammation of the wound is aggravated and healing of the wound is delayed by accumulation of generated bacterial fragments caused by the bactericide killing the bacteria. Therefore, the disclosure breaks through inherent antibacterial thought of anti-infective dressings; and can realize anti-infection to the wound bacteria through selection and adaption of a structure and surface energy of the dressing, and is especially suitable for pre-wound anti-infection and low bacterial concentration situations, and the dressing can be changed in time in use, so as to improve adhesion efficiency to the bacterial. The wound bacteria can be transferred and removed by changing dressings, thus reducing concentration of the wound bacteria and further reducing a risk of wound infection. In addition, the product also has excellent anti-adhesive performance, which can effectively prevent secondary injury to the wound in dressing changing and significantly promote healing of the wound.

The polyolefin on the surface of micron-sized porous membrane is compounded by grafting or coating on a substrate surface of the micron-sized porous membrane (for example, the micron-sized porous membrane substrate is prepared first, and the micron-sized porous membrane substrate is a micron-sized porous fiber membrane substrate; then a layer of polyolefin material is compounded on its surface by chemical modification or physical coating), or the micron-sized porous polyolefin fiber membrane is directly obtained by polyolefin spinning. The micron-sized porous polyolefin fiber membrane forms nonspecific adhesion to the bacteria on the wound by adaptability of its own surface energy and the structure of the fiber membrane to the surface energy of the bacterial, so as to realize the anti-infection for the bacteria and the anti-adhesion to the wound. The polyolefin is poly-4-methyl-1 pentene or a mixture of poly-4-methyl-1 pentene and polyethylene or polypropylene, with a mass fraction of poly-4-methyl-1 pentene to be 30% to 100%, preferably 50% to 100%, more preferably 80% to 100%. It is shown in the disclosure that the fiber porous membrane containing poly-(4-methyl-1-pentene) has excellent adhesion to the bacteria, good biocompatibility, and is not easy to adhere to wound tissues and has excellent anti-infective performance when used as a wound dressing, which is superior to that of similar dressings in the market. It can be seen that in this disclosure, the high-efficiency anti-infective and anti-adhesive performance can be achieved through targeted selection and design of the material and the surface structure of the porous membrane.

A fiber diameter of the micron porous polyolefin fiber membrane is 0.1 to 15 µm, with a pore diameter of 1 to 10 µm. Research results of the disclosure show that with such parameters, the adhesive performance to the bacteria and the anti-adhesive performance for tissues are optimal. A thickness of the micron porous polyolefin fiber membrane is 0. 1 to 1 mm.

A preparation method of a micron-sized porous polyolefin fiber membrane includes: preparing a polyolefin spinning solution with a mass fraction of 1% to 7%, and then carrying out electrostatic spinning so as to receive and obtain the micron-sized porous polyolefin fiber membrane with an aluminum foil. A solvent of the polyolefin spinning solution includes cyclohexane and N, N dimethylformamide.

The solvent of the polyolefin spinning solution also includes acetone. Mass fractions of cyclohexane, N, N dimethylformamide and acetone in the spinning solution are in ranges of 70% to 80%, 10% to 20%, and 0% to 20% respectively. A mass ratio of cyclohexane, N, N dimethylformamide and acetone is 4: 1: 1 to 1: 1: 1.

An extrusion speed of the electrostatic spinning is 1 mL/h to 7 mL/h; a distance for receiving the fiber is 8 to 12 cm; a voltage for the electrostatic spinning is +18 kV to +20 kV, or -1 kV to -3 kV; and a spinning temperature is 45°C to 60°C.

Specifically, the preparation method of the micron-sized porous polymethylpentene fiber membrane includes following steps.
(1) Polyolefin masterbatch is dried at 60°C for 8h to remove free water on its surface;
(2) A polyolefin solution is prepared according to following mass percentages:

| | |
|---|---|
| Polymethylpentene | 1 % to 7 % |
| cyclohexane | 70 % to 80 % |
| N,N- dimethylformamide | 10% to 20% |
| acetone | 0 % to 20 % |

These solvents are uniformly mixed, then the dried polyolefin masterbatch in step (1) is added into the mixed solvents, and magnetically stirred under a heating condition to dissolve the polyolefin masterbatch, with dissolving time of the polyolefin masterbatch of 6 to 8h and a dissolving temperature of 45 to 60°C.
(3) The electrospinning solution is spinned on an electrospinning device, and micron fibers are collected so as to obtain an anti-infective wound dressing.

The micron-sized porous polyolefin fiber membrane prepared according to the disclosure is with a strength of 2 to 10MPa and an elongation at break of 25% to 85%. Compared with a petrolatum gauze in Comparative Embodiment 1, its anti-adhesion performance is better, and a peeling force per unit area of a simulated wound is about 0.15 N lower than that of the petrolatum gauze.

### Embodiment 1

A preparation method of an anti-infective and anti-adhesive wound dressing based on nonspecific adhesion to bacteria is provided in this embodiment, which includes the following steps.

Step (1): pretreatment of materials in which poly-4-methyl-1 pentene is dried at 60°C for 4 hours to remove surface moisture.

Step (2): preparation of a spinning solution in which cyclohexane and N, N dimethylformamide with a mass fraction of 80%: 20% are mixed into a binary solvent, then poly-4-methyl-1 pentene with a mass fraction of 3% is added into the mixed solvent, which is magnetically stirred at 60°C for 8h until it dissolves.

Step (3): Electrostatic spinning in which the spinning solution is added into an injector and spinning at 45°C. it is provided with an extrusion speed of 1 .086 ml/h, a receiving distance of 20 cm, a voltage of +20kV and -3kV, a 22G needle, and a aluminum foil for receiving a fiber membrane.

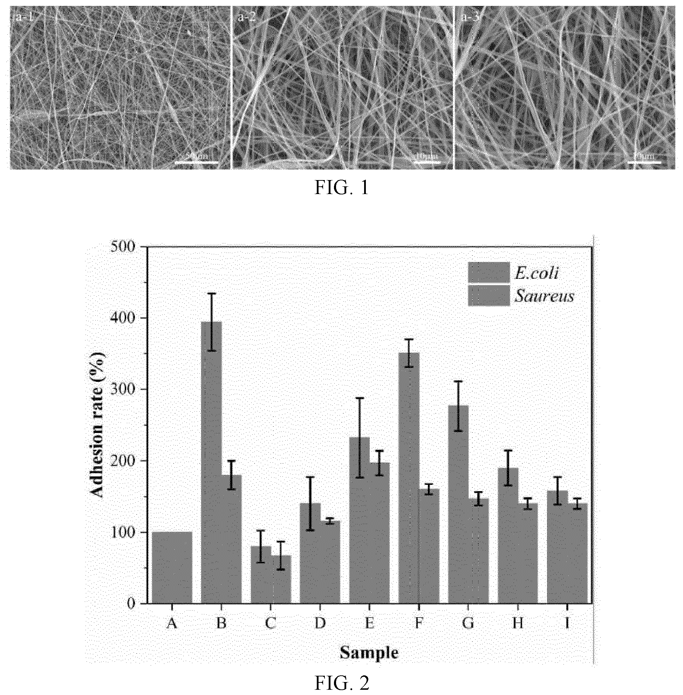

Referring to FIG. 1, the poly-4-methyl-1 pentene fiber membrane prepared in this embodiment is with an average pore diameter of about 2.33 µm and a fiber diameter of 0.5 µm.

### Embodiments 2 to 3 (not according to the invention)

Preparation methods of an anti-infective and anti-adhesive wound dressing based on nonspecific adhesion to bacteria according to Embodiments 2 to 3 are different from Embodiment 1 in that poly-4-methyl-1 pentene in Step (2) is replaced with polyethylene and polypropylene respectively. Other aspects are substantially the same as Embodiment 1, which will not be repeatedly described here again.

### Embodiment 4

A preparation method of an anti-infective and anti-adhesive wound dressing based on nonspecific adhesion to bacteria according to Embodiment 4 is different from Embodiment 1 in that poly-4-methyl-1 pentene in step (2) is replaced with 2% of poly-4-methyl-1 pentene and 1% of polyethylene respectively. Other aspects are substantially the same as Embodiment 1, which will not be repeatedly described here again.

### Embodiments 5 to 8

Preparation methods of an anti-infective and anti-adhesive wound dressing based on nonspecific adhesion to bacteria according to Embodiments 5 to 8 are different from Embodiment 1 in that the mass fraction of polymethylpentene in Step (2) is changed to 4%, 5%, 6% and 7% in turn. Other aspects are substantially the same as Embodiment 1, which will not be repeatedly described here again.

**Table 1 parameters and performance test results of fiber membranes of Embodiments 1, and 5 to 8**

| Embodiments | Fiber Diameter (µm) | Pore Diameter (µm) | Anti-adhesion to Wound (N) | Ability to Adhere to Bacteria (%) | Rat Mortality |
|---|---|---|---|---|---|
| 1 | 0.5 | 2.33 | 0.146 | 394 | / |
| 5 | 3 | 5.35 | 0.148 | 351 | / |
| 6 | 7 | 6.85 | 0.151 | 276 | / |
| 7 | 9 | 9.20 | 0.153 | 190 | / |
| 8 | 15 | 12.42 | 0.187 | 158 | / |

As can be seen from Table 1, Embodiments 1, and 5 to 8 are with fiber dressings prepared from poly-4-methyl-1 pentene of different mass fractions, with different fiber diameters and pore diameters. When the fiber diameter is too large, the adhesion of the poly-4-methyl-1 pentene fiber membrane to the bacteria is significantly reduced, and the anti-adhesion to the wound surface is poor (an adhesion force becomes large). This may be because with a proper fiber diameter and pore diameter being provided for a poly-4-methyl-1 pentene base material, good adaptability with a diameter of the bacteria and diameters of human tissue cells can be formed, thus significantly reducing the adhesion to human tissue cells while ensuring high adhesion to bacteria cells.

### Comparative Embodiment 1

An anti-infective and anti-adhesive wound dressing based on nonspecific adhesion to bacteria is provided, with acetate fiber fabric as a substrate and with hydrophobic dialkyl carbamoyl chloride being impregnated on a surface thereof.

Reference is made to FIG. 2, which shows bacterial adhesion performance of the anti-infective dressings prepared in Comparative Embodiment 1 and Embodiments 1 to 8 (Escherichia coli is indicated by E .coli and Staphylococcus is indicated by Saureus in the figure). In this figure, A indicates the anti-infective dressing of Comparative Embodiment 1, and B to I correspondingly indicate

Embodiments 1 to 8. It can be seen that the bacterial adhesion of Embodiment 1 is the highest. It can be seen from B and F to H that the bacterial adhesion of the anti-infective dressing prepared from poly-4-methyl-1 pentene is higher than that of polyethylene and polypropylene.

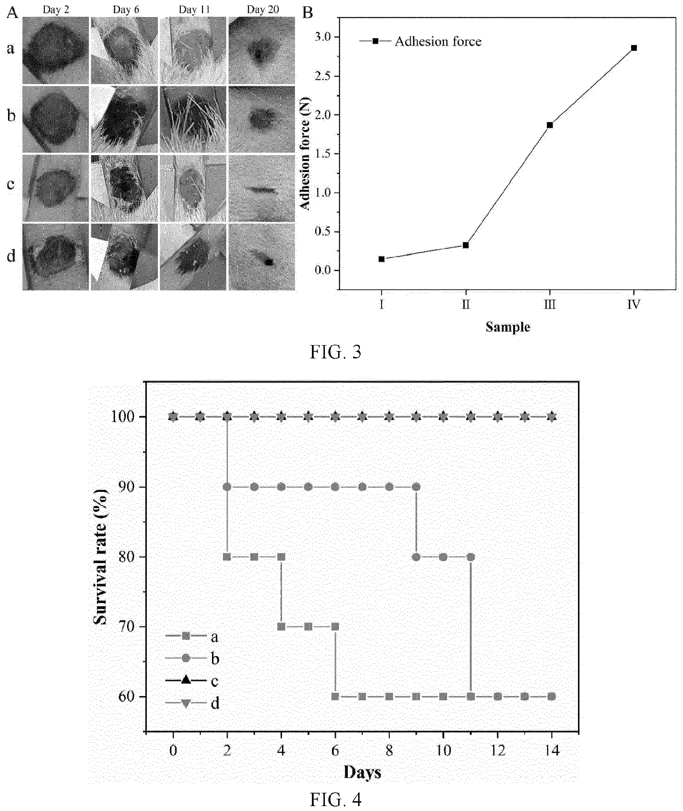

Reference is made to FIG. 3, in which panel A illustrates healing processes of infected wounds of rats in four groups, with an initial infection condition in which about 3 million bacteria are coated on wound sites of rats, and a bacterial infection model is derived after continuous feeding for 1 day. Group a is a bacterial infected group (with no treatment), group b is an infected and treated group (with the anti-infective dressing in Comparative Embodiment 1), group c is an infected and treated group (with the anti-infective dressing in this disclosure), and group d is blank control group d (with no infection). It can be seen that the blank control group d has shortest wound healing time, followed by the group c treated with the anti-infective dressing in this disclosure, the group b treated with the anti-infective dressing in the Comparative Embodiment 1, and the group a with on treatment, which indicates that the anti-infective dressing in this disclosure can effectively promote healing of infected wounds, and has better performance than that of the anti-infective dressing in Comparative Embodiment 1.

Panel B illustrates simulated wound peeling strength tests of the anti-infective dressing in this disclosure and several dressings in Comparative Embodiment 1 (I: the anti-infective dressing in this disclosure; II: a petrolatum gauze (which is obtained by coating petrolatum on an ordinary gauze, which is a most commonly used anti-adhesive gauze on the market at present, also called oil gauze); III: the anti-infective dressing in the Comparative Embodiment 1; and IV: an ordinary cotton gauze swab), with peeling forces per unit area of 0.146 N, 0.323 N, 1.871 N and 2.857 N, respectively. It can be seen that the anti-infective dressing in this disclosure is easy to peel off from the wound surface, thus avoiding the secondary injury of the wound surface in dressing changing.

FIG. 4 shows survival rates of rats in four groups in treatment of infected wounds. Group a is a bacterial infected group (with no treatment), group b is an infected and treated group (with the anti-infective dressing in Comparative Embodiment 1), group c is an infected and treated group (with the anti-infective dressing in this disclosure), and group d is blank control group d (with no infection). It can be seen that a survival rate of a common infected group is only 60% after 6 days, and a survival rate of an infected group treated with the dressing treatment in Comparative Embodiment 1 is only 60% after 11 days, while survival rates of the group treated with the dressing in this disclosure and of the blank control group are still 100% until 14 days of wound healing, and the dressing in this disclosure has excellent therapeutic performance.

To sum up, the anti-infective and anti-adhesive wound dressing based on nonspecific adhesion to bacteria according to the disclosure is a micron-sized porous membrane material with polyolefin contained in its surface, with no bactericide being added. In this way, nonspecific adhesion to the bacteria on the wound can be formed by adaptability of the surface energy of the micron-sized porous membrane and the structure of the porous membrane to the surface energy of the bacterial, preventing the bacteria from penetrating into wound tissues and thus realizing the anti-infection for the bacteria and the anti-adhesion to the wound. Therefore, compared with general anti-infective dressings, no antibacterial ingredient is required to be added, and the dressing of the present disclosure has good biocompatibility and thus no negative impact on the wound to inhibit its recovery, which breaks through inherent antibacterial thought of anti-infective dressings; and high-efficiency anti-infective and anti-adhesive performance can be achieved through targeted selection and design of a material and a surface structure of the porous membrane.

## Claims

1. An anti-infective and anti-adhesive wound dressing based on nonspecific adhesion to bacteria, comprising a micron-sized porous membrane with polyolefin contained in a surface thereof and does not contain any bactericide, wherein the micron-sized porous membrane forms nonspecific adhesion to bacteria on a wound by adaptability of surface energy and a structure of the porous membrane to surface energy of the bacterial, so as to realize the anti-infection for the bacteria and the anti-adhesion to the wound;
wherein the polyolefin is polymethylpentene.

2. The anti-infective and anti-adhesive wound dressing based on nonspecific adhesion to bacteria according to claim 1, wherein the polyolefin on the surface of micron-sized porous membrane is compounded by grafting or coating on a substrate surface of the micron-sized porous membrane, or the micron-sized porous polyolefin fiber membrane is directly obtained by polyolefin spinning.

3. The anti-infective and anti-adhesive wound dressing based on nonspecific adhesion to bacteria according to claim 2, wherein the micron-sized porous membrane substrate is a micron-sized porous fiber membrane substrate.

4. The anti-infective and anti-adhesive wound dressing based on nonspecific adhesion to bacteria according to any one of claims 1 to 3, wherein a pore diameter of the micron-sized porous membrane is 1 to 10 µm.

5. The anti-infective and anti-adhesive wound dressing based on nonspecific adhesion to bacteria according to claim 3, wherein fiber diameters of the micron-sized porous fiber membrane substrate and the micron-sized porous polyolefin fiber membrane are 0. 1 to 15 µm.

6. The anti-infective and anti-adhesive wound dressing based on nonspecific adhesion to bacteria according to claim 2, wherein a preparation method of the micron-sized porous polyolefin fiber membrane comprises: preparing a polyolefin spinning solution with a mass fraction of 1% to 7%, and then carrying out electrostatic spinning so as to receive and obtain the micron-sized porous polyolefin fiber membrane with an aluminum foil; wherein a solvent of the polyolefin spinning solution includes cyclohexane and N, N dimethylformamide.

7. The anti-infective and anti-adhesive wound dressing based on nonspecific adhesion to bacteria according to claim 6, wherein the solvent of the polyolefin spinning solution further comprises acetone.

8. The anti-infective and anti-adhesive wound dressing based on nonspecific adhesion to bacteria according to claim 7, wherein mass fractions of cyclohexane, N, N dimethylformamide and acetone in the spinning solution are in ranges of 70% to 80%, 10% to 20%, and 0% to 20% respectively.

9. The anti-infective and anti-adhesive wound dressing based on nonspecific adhesion to bacteria according to claim 6, wherein an extrusion speed of the electrostatic spinning is 1 mL/h to 7 mL/h; a distance for receiving the fiber is 8 to 12 cm; a voltage for the electrostatic spinning is +18 kV to 20 kV, or -1 kV to -3 kV; and a spinning temperature is 45 °C to 60°C.

## Patentansprüche

1. Antiinfektiöser und antiadhäsiver Wundverband auf der Basis von unspezifischer Adhäsion an Bakterien, umfassend eine poröse Membran in Mikrongröße mit Polyolefin, das in einer Oberfläche davon enthalten ist, und die keinerlei Bakterizid enthält, wobei die poröse Membran in Mikrongröße durch Anpassbarkeit von Oberflächenenergie und eine Struktur der porösen Membran zur Oberflächenenergie der Bakterien eine unspezifische Adhäsion an Bakterien auf einer Wunde bildet, um die Antiinfektiösität für die Bakterien und die Antiadhäsion an der Wunde umzusetzen;
wobei das Polyolefin Polymethylpenten ist.

2. Antiinfektiöser und antiadhäsiver Wundverband auf der Basis von unspezifischer Adhäsion an Bakterien nach Anspruch 1, wobei das Polyolefin auf der Oberfläche der porösen Membran in Mikrongröße durch Pfropfen oder Beschichten auf eine Substratoberfläche der porösen Membran in Mikrongröße compoundiert wird oder die poröse Polyolefinfaser-Membran in Mikrongröße direkt durch Polyolefinspinnen erhalten wird.

3. Antiinfektiöser und antiadhäsiver Wundverband auf der Basis von unspezifischer Adhäsion an Bakterien nach Anspruch 2, wobei das poröse Membran-Substrat in Mikrongröße ein poröses Faser-Membran-Substrat in Mikrongröße ist.

4. Antiinfektiöser und antiadhäsiver Wundverband auf der Basis von unspezifischer Adhäsion an Bakterien nach einem der Ansprüche 1 bis 3, wobei ein Porendurchmesser der porösen Membran in Mikrongröße 1 bis 10 µm beträgt.

5. Antiinfektiöser und antiadhäsiver Wundverband auf der Basis von unspezifischer Adhäsion an Bakterien nach Anspruch 3, wobei Faserdurchmesser des porösen Faser-Membran-Substrats in Mikrongröße und der porösen Polyolefinfaser-Membran in Mikrongröße 0,1 bis 15 µm betragen.

6. Antiinfektiöser und antiadhäsiver Wundverband auf der Basis von unspezifischer Adhäsion an Bakterien nach Anspruch 2, wobei ein Herstellungsverfahren der porösen Polyolefinfaser-Membran in Mikrongröße umfasst: Herstellen einer Polyolefinspinnlösung mit einem Massenanteil von 1 % bis 7 % und dann Ausführen eines elektrostatischen Spinnens, um die poröse Polyolefinfaser-Membran in Mikrongröße mit einer Aluminiumfolie aufzunehmen und zu erhalten; wobei ein Lösungsmittel der Polyolefinspinnlösung Cyclohexan und N,N-Dimethylformamid beinhaltet.

7. Antiinfektiöser und antiadhäsiver Wundverband auf der Basis von unspezifischer Adhäsion an Bakterien nach Anspruch 6, wobei das Lösungsmittel der Polyolefinspinnlösung weiterhin Aceton umfasst.

8. Antiinfektiöser und antiadhäsiver Wundverband auf der Basis von unspezifischer Adhäsion an Bakterien nach Anspruch 7, wobei Massenanteile von Cyclohexan, N,N-Dimethylformamid und Aceton in der Spinnlösung in Bereichen von 70 % bis 80 %, 10 % bis 20 % bzw. 0 % bis 20 % liegen.

9. Antiinfektiöser und antiadhäsiver Wundverband auf der Basis von unspezifischer Adhäsion an Bakterien nach Anspruch 6, wobei eine Extrusionsgeschwindigkeit des elektrostatischen Spinnens 1 mL/h bis 7 mL/h beträgt; ein Abstand zum Aufnehmen der Faser 8 bis 12 cm beträgt; eine Spannung für das elektrostatische Spinnen +18 kV bis 20 kV oder -1 kV bis -3 kV beträgt und eine Spinntemperatur 45 °C bis 60 °C beträgt.

## Revendications

1. Pansement pour plaie anti-infectieux et anti-adhésif basé sur une adhésion non spécifique à des bactéries, comprenant une membrane poreuse de taille micrométrique avec une polyoléfine contenue dans une surface de celle-ci et ne contenant aucun bactéricide, la membrane poreuse de taille micrométrique formant une adhésion non spécifique à des bactéries sur une plaie par adaptabilité de l'énergie de surface et d'une structure de la membrane poreuse à l'énergie de surface de la bactérie, de façon à réaliser l'anti-infection pour les bactéries et l'anti-adhésion à la plaie ;
la polyoléfine étant du polyméthylpentène.

2. Pansement pour plaie anti-infectieux et anti-adhésif basé sur une adhésion non spécifique à des bactéries selon la revendication 1, dans lequel la polyoléfine sur la surface de la membrane poreuse de taille micrométrique est composée par greffage ou revêtement sur une surface de substrat de la membrane poreuse de taille micrométrique, ou la membrane de fibre polyoléfinique poreuse de taille micrométrique est directement obtenue par filage de polyoléfine.

3. Pansement pour plaie anti-infectieux et anti-adhésif basé sur une adhésion non spécifique à des bactéries selon la revendication 2, dans lequel le substrat de membrane poreuse de taille micrométrique est un substrat de membrane de fibre poreuse de taille micrométrique.

4. Pansement pour plaie anti-infectieux et anti-adhésif basé sur une adhésion non spécifique à des bactéries selon l'une quelconque des revendications 1 à 3, dans lequel un diamètre de pore de la membrane poreuse de taille micrométrique va de 1 à 10 µm.

5. Pansement pour plaie anti-infectieux et anti-adhésif basé sur une adhésion non spécifique à des bactéries selon la revendication 3, dans lequel les diamètres de fibre du substrat de membrane de fibre poreuse de taille micrométrique et de la membrane de fibre polyoléfinique poreuse de taille micrométrique vont de 0,1 à 15 µm.

6. Pansement pour plaie anti-infectieux et anti-adhésif basé sur une adhésion non spécifique à des bactéries selon la revendication 2, dans lequel un procédé de préparation de la membrane de fibre polyoléfinique poreuse de taille micrométrique comprend : la préparation d'une solution de filage de polyoléfine avec une fraction massique de 1 % à 7 %, puis la réalisation d'un filage électrostatique de façon à recevoir et obtenir la membrane de fibre polyoléfinique poreuse de taille micrométrique avec une feuille d'aluminium ; un solvant de la solution de filage de polyoléfine comprenant du cyclohexane et du N,N-diméthylformamide.

7. Pansement pour plaie anti-infectieux et anti-adhésif basé sur une adhésion non spécifique à des bactéries selon la revendication 6, dans lequel le solvant de la solution de filage de polyoléfine comprend en outre de l'acétone.

8. Pansement pour plaie anti-infectieux et anti-adhésif basé sur une adhésion non spécifique à des bactéries selon la revendication 7, dans lequel les fractions massiques de cyclohexane, de N,N-diméthylformamide et d'acétone dans la solution de filage sont dans des plages de 70 % à 80 %, 10 % à 20 %, et 0 % à 20 % respectivement.

9. Pansement pour plaie anti-infectieux et anti-adhésif basé sur une adhésion non spécifique à des bactéries selon la revendication 6, dans lequel une vitesse d'extrusion du filage électrostatique va de 1 mL/h à 7 mL/h ; une distance pour recevoir la fibre va de 8 à 12 cm ; une tension pour le filage électrostatique va de +18 kV à 20 kV, ou de -1 kV à -3 kV ; et une température de filage va de 45 °C à 60 °C.
